(19) Europäisches Patentamt
European Patent Office
Office européen des brevets

(11) Veröffentlichungsnummer: **0 004 957**
**A2**

# (12) EUROPÄISCHE PATENTANMELDUNG

(21) Anmeldenummer: **79101156.2**

(22) Anmeldetag: **17.04.79**

(51) Int. Cl.²: **C 07 C 53/36**
C 07 C 53/32, C 07 C 69/63
C 07 C 103/34, C 10 M 3/00

(30) Priorität: **26.04.78 CH 4504/78**

(43) Veröffentlichungstag der Anmeldung:
**31.10.79 Patentblatt 79/22**

(84) Benannte Vertragsstaaten:
**BE DE FR GB IT NL**

(71) Anmelder: **CIBA-GEIGY AG**
**Patentabteilung Postfach**
**CH-4002 Basel(CH)**

(72) Erfinder: **Steiner, Eginhard, Dr.**
**Obere Hofackerstrasse 3**
**CH-4414 Füllinsdorf(CH)**

(72) Erfinder: **Schmidt, Andreas, Dr.**
**Hubackerweg 32**
**CH-4153 Reinach(CH)**

## (54) Chlorierte Derivate der Buttersäure und deren Verwendung als Schmiermittelzusätze.

(57) Verbindungen der allgemeinen Formel I

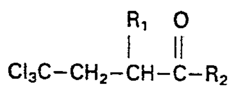

(I),

worin

| | |
|---|---|
| $R_1$ | Chlor oder $-CH_2-CCl_3$ ist, und |
| $R_2$ | $-N(R_3)R_4$, $-OH \cdot N(R_3)(R_4)R_5$ oder $-OR_6$ ist, wobei |
| $R_3$ | $C_1$-$C_{24}$ Alkyl ist welches gegebenenfalls ein- oder mehrmals mit einem Sauerstoffatom unterbrochen sein kann und |
| $R_4$ und $R_5$ | unabhängig voneinander Wasserstoff oder $C_1$-$C_{24}$ Alkyl bedeuten, wobei $R_3$ und $R_4$ zusammen mindestens 8 C-Atome besitzen, und |
| $R_6$ | $C_8$-$C_{20}$ Alkyl, Phenyl oder $C_7$-$C_{15}$ Alkylphenyl, eine Gruppe der Formel $-(A)R_7$ oder $-CH_2CH_2-N(R_{10})-CH_2CH_2-O-C(O)-CH(R_1)CH_2CCl_3$ ist, worin |
| $R_7$ | einen Rest der Formel $-(R_8)C(R_9)-O-C(O)-CH(R_1)-CH_2-CCl_3$ bedeutet, wobei |
| $R_8$ und $R_9$ | unabhängig voneinander Wasserstoff, Methyl oder Aethyl sind, und $R_1$ die oben angegebene Bedeutung hat, und |
| A | eine gegebenenfalls mit einem oder zwei Resten $R_7$ oder einer oder zwei Methyl- oder Aethylgruppen substituierte $C_1$-$C_7$ Alkylengruppe ist, welche gegebenenfalls durch -O- unterbrochen sein kann, und |
| $R_{10}$ | $C_4$-$C_{20}$ Alkyl bedeutet. |

EP 0 004 957 A2

0004957

CIBA-GEIGY AG
Basel Schweiz

3-11686/ZFO/1+2

BEZEICHNUNG GEÄNDERT
siehe Titelseite

Schmiermittelzusätze

Die vorliegende Erfindung betrifft neue chlorierte Derivate der Buttersäure, deren Verwendung als Schmiermittelzusätze, deren Herstellung, sowie die mit den neuen Verbindungen ausgerüsteten Schmiermittel.

Mineralischen und synthetischen Schmierstoffen werden im allgemeinen verschiedene Zusatzstoffe zur Verbesserung ihrer Gebrauchseigenschaften beigegeben. Insbesondere besteht ein Bedarf an Additiven, welche die zu schmierenden Vorrichtungen vor Reibungsabnützung schützen sollen. An solche Verschleissinhibitoren wird die Anforderung gestellt, dass sie das Lasttragevermögen des Schmierstoffs erhöhen und nicht korrodierend auf die zu schützenden Metallteile wirken. Aus der DE-AS Nr. 1,033,355 ist bekannt, α,α,γ,γ-Tetrachlorbuttersäure als Zusatz für Schmiermittel zu verwenden. Diese Verbindung wirkt allerdings korrodierend, was ihre technische Verwendbarkeit stark einschränkt.

Es sind in FR Pat. Nr. 1,242,382 auch Niederalkylester der $\alpha,\alpha,\gamma,\gamma$-Tetrachlorbuttersäure beschrieben worden, deren Wirksamkeit jedoch den hohen Anforderungen nicht gewachsen ist. Ferner sind auch Ammoniumsalze von halogenierten Carbonsäuren, wie z.B. der $\alpha,\alpha,\gamma$-Trichlorbuttersäure im US Pat. Nr. 3,785,977 als Schmiermittelzusätze vorgeschlagen worden. Solche Verbindungen haben jedoch den Nachteil, dass sie hydrolyseanfällig sind und von eingedrungenem Wasser gelöst werden, was ein Nachlassen ihrer Verschleisshemmenden und Hochdruck-Eigenschaften zur Folge hat.

Es wurde nun eine Klasse von chlorierten Buttersäurederivaten gefunden, welche eine ausgezeichnete Wirksamkeit als Hochdruck- und Antiwear-Additive in Schmiermitteln und Schneidölen entfalten, und neben geringer Flüchtigkeit auch minimale Wasserlöslichkeit aufweisen, wodurch in Schmiersystemen eine Reduktion an Wirksubstanz durch Wasseraustrag kaum auftritt. Besonders wertvoll werden die neuen Substanzen durch die Kombination der erwähnten Eigenschaften mit ihrer ausgezeichneten Korrosionsschutz-Wirkung gemacht.

Die neuen Verbindungen entsprechen der allgemeinen Formel I

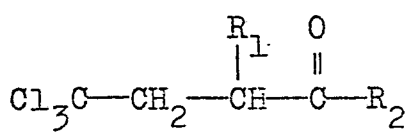

(I) ,

worin

$R_1$ Chlor oder $-CH_2-CCl_3$ ist, und

$R_2$ $-N(R_3)R_4$, $-OH\cdot N(R_3)(R_4)R_5$ oder $-OR_6$ ist, wobei

$R_3$ $C_1$-$C_{24}$ Alkyl, welches gegebenenfalls ein- oder mehrmals durch Sauerstoff unterbrochen sein kann,

$R_4$ und $R_5$ unabhängig voneinander Wasserstoff oder $C_1$-$C_{24}$ Alkyl bedeuten, wobei $R_3$ und $R_4$ zusammen mindestens 8 C-Atome besitzen oder $R_3$, und

$R_6$ $C_8$-$C_{20}$ Alkyl, Phenyl oder $C_7$-$C_{15}$ Alkylphenyl, eine Gruppe der Formel $-(A)R_7$ oder $-CH_2CH_2-N(R_{10})-CH_2CH_2-O-C(O)-CH(R_1)CH_2CCl_3$ ist, worin

$R_7$ einen Rest der Formel $-(R_8)C(R_9)-O-C(O)-CH(R_1)-CH_2-CCl_3$ bedeutet, wobei

$R_8$ und $R_9$ unabhängig voneinander Wasserstoff, Methyl oder Aethyl sind, und $R_1$ die oben angegebene Bedeutung hat, und

A eine gegebenenfalls mit einem oder zwei Resten $R_7$ oder einer oder zwei Methyl- oder Aethylgruppen substituierte $C_1$-$C_7$ Alkylengruppe ist, welche gegebenenfalls durch -O- unterbrochen sein kann, und

$R_{10}$ $C_4$-$C_{20}$ Alkyl bedeutet.

$R_3$, $R_4$ und $R_5$ sind als $C_1$-$C_{24}$ und bevorzugt $C_4$-$C_{18}$ Alkyl beispielsweise Methyl, Aethyl, n-Propyl, Isopropyl, n-Butyl, sec.Butyl, tert.Butyl, n-Pentyl, tert.Pentyl, Isopentyl, Neopentyl, n-Hexyl, Isohexyl, Heptyl, Octyl, 1,1,3,3-Tetramethylbutyl, 2-Aethylhexyl, n-Octyl, Decyl, Undecyl, 1,1,3,3,5,5-Hexamethylhexyl. n-Dodecyl, Tridecyl, Tetradecyl, Pentadecyl, Hexadecyl, Heptadecyl, Octadecyl, Nonadecyl, Eicosyl, Heneicosyl, Docosyl, Tricosyl oder Tetracosyl, Doekayloseäthyl, Isotridecyloseyodenyl, Isononyloseypropyl-. Bevorzugt werden Verbindungen, in welchen mindestens einer der Reste $R_3$, $R_4$ oder $R_5$ $C_8$-$C_{18}$ Alkyl ist. Bevorzugt werden aber auch Verbindungen, in welchen $R_4$ bzw. $R_4$ und $R_5$ Wasserstoff sind.

$R_6$ kann als $C_8$-$C_{24}$ Alkyl z.B. n-Octyl, 1,1,3,3-Tetramethylbutyl, 2-Aethylhexyl, Nonyl, Decyl, n-Dodecyl, 1,1,3,3,5,5-Hexamethylhexyl, Tridecyl, Tetradecyl, Pentadecyl, Hexadecyl, Heptadecyl, Octadecyl, Octadecyl, Nonadecyl, Eicosyl, Heneicosyl, Docosyl, Tricosyl oder Tetracosyl sein. Bevorzugt ist $R_6$ als Alkylgruppe eine solche mit 8-18 C-Atomen.

$R_6$ ist als Alkylphenyl mit 7-15 C-Atomen z.B. 2,4-Di-t.butylphenyl, 2,6-Di-t.butyl-4-methylphenyl oder p-Nonylphenyl.

Bei verschiedenen für $R_4$, $R_5$ und $R_6$ oben beispielhaft erwähnten langkettigen Alkylgruppen sind keine reinen Isomeren erwähnt. Der Grund dafür liegt einerseits darin, dass sich Isomerengemische als Schmiermittelzusätze ebensogut eignen, wie reine Isomere und anderseits darin, dass die zur Herstellung der Verbindungen der Formel I verwendeten Alkohole bzw. Amine oftmals als Isomerengemische oder gar als Gemische von Verbindungen, welche Alkylgruppen verschiedener Länge enthalten, im Handel angeboten werden. Als Beispiel sei hier Primen 81-R (Röhm und Haas, USA) erwähnt, welches ein Gemisch primärer $C_{12}$-$C_{15}$ Alkylamine verschiedener Isomerie darstellt.

Ist $R_6$ eine Gruppe der Formel -(A)$R_7$, so handelt es sich bei A als $C_1$-$C_8$ Alkylengruppe, welche gegebenenfalls durch -O- unterbrochen sein kann, beispielsweise um Methylen, Dimethylen, Trimethylen, Tetramethylen, Hexamethylen, Heptamethylen oder um 3,6-Dioxa-heptamethylen, bevorzugt um Methylen, Dimethylen oder um 3,6-Dioxa-heptamethylen. Ist A eine mit einer oder zwei Methyl- oder Aethylgruppen substituiertes Alkylen, so sind beispielhaft

1,1-Dimethyldimethylen, 1-Methyl-1-äthyldimethylen oder Dimethylmethylen zu nennen. Mit Resten $R_7$ substituierte Alkylengruppen A sind z.B. $-CH(R_7)-$, $-CH_2CH_2CH(R_7)-CH_2-$ oder $-CH_2-C(R_7)_2-$.

$R_8$ und $R_9$ bedeuten Aethyl, bevorzugt Methyl und besonders bevorzugt Wasserstoff. Bevorzugt werden Verbindungen, in welchen $R_8$ und $R_9$ die gleiche Bedeutung haben.

$R_{10}$ kann als $C_4$-$C_{20}$ Alkyl beispielsweise n-Butyl, sec.Butyl, tert.-Butyl, n-Pentyl, Isopentyl, Neopentyl, Isohexyl, n-Hexyl, n-Octyl, 2-Aethylhexyl, Decyl, Dodecyl, Tetradecyl, Octadecyl oder Eicosyl sein.

Bevorzugt werden Verbindungen der Formel I, worin

$R_1$ Chlor oder $-CH_2-CCl_3$ bedeutet, und

$R_2$ $-N(R_3)R_4$, $-OH\cdot N(R_3)(R_4)R_5$ oder $-OR_6$ ist, und

$R_3$ $C_4$-$C_{18}$ Alkyl ist, und

$R_4$ und $R_5$ unabhängig voneinander Wasserstoff oder $C_4$-$C_{18}$ Alkyl bedeuten, wobei $R_3$ und $R_4$ zusammen mindestens 8 C-Atome besitzen, und

$R_6$ $C_8$-$C_{18}$ Alkyl oder $C_7$-$C_{15}$ Alkylphenyl oder eine Gruppe der Formel $-(A)R_7$ ist, worin

$R_7$ einen Rest der Formel $-(R_8)C(R_9)-O-C(O)-CH(R_1)-CH_2-CCl_3$ bedeutet, wobei

$R_8$ und $R_9$ unabhängig voneinander Wasserstoff, Methyl oder Aethyl sind, und $R_1$ die oben angegebene Bedeutung hat und

A eine gegebenenfalls mit einem oder zwei Resten $R_7$ oder einer oder zwei Methyl- oder Aethylgruppen substituierte $C_1$-$C_5$ Alkylengruppe oder eine 3,6-Dioxaheptamethylengruppe bedeutet.

Besonders bevorzugt werden Verbindungen der Formel I, worin

$R_1$ Chlor oder $-CH_2-CCl_3$ bedeutet, und

$R_2$ $-N(R_3)(R_4$, $-OH \cdot N(R_3)(R_4)R_5$ oder $-OR_6$ ist, und

$R_3$ $C_4-C_{18}$ Alkyl ist, und

$R_4$ und $R_5$ unabhängig voneinander Wasserstoff oder $C_4-C_{18}$ Alkyl bedeuten, wobei $R_3$ und $R_4$ zusammen mindestens 8 C-Atome besitzen, und

$R_6$ $C_8-C_{18}$ Alkyl, $C_7-C_{15}$ Alkylphenyl oder eine Gruppe der Formel $-(A)R_7$ ist, worin

$R_7$ einen Rest der Formel $-CH_2-O-C(O)-CH(R_1)-CH_2-CCl_3$ bedeutet, wobei $R_1$ die oben angegebene Bedeutung hat und

A $C_1-C_5$ Alkylen, 3,6-Dioxaheptamethylen oder eine der Gruppen der Formeln $-CH(R_7)-$, $-CH_2CH_2CH(R_7)-CH_2-$ oder $-CH_2-C(R_7)_2-$ bedeutet, worin $R_7$ die oben angegebene Bedeutung hat.

Beispiele für Verbindungen der Formel I sind:

1) 2,4,4,4-Tetrachlorbuttersäure-N-octadecylamid

2) Bis-(2',2',2'-trichloräthyl)-essigsäure-N-dodecylamid

3) Das

a) Octadecylammoniumsalz der (i)/(ii)

b) Dioctylammoniumsalz der (i)/(ii)

c) Di-n-butylammoniumsalz der (i)/(ii)

d) Tri-n-butylammoniumsalz der (i)/(ii)

e) Dodecylammoniumsalz der (i)/(ii)

f) 1,1,3,3-Tetramethylbutylammoniumsalz der (i)/(ii)

0004957

g) Gemisch der Eicosyl-bis-Docosylammoniumsalze der (i)/(ii)

(i) 2,4,4,4-Tetrachlorbuttersäure oder

(ii) Bis-(2',2',2'-trichloräthyl)-essigsäure

4) 2,4,4,4-Tetrachlorbuttersäure-octadecylester

5) Bis-(2',2',2'-trichloräthyl)-essigsäure-dodecylester

6) Bis-(2',2',2'-trichloräthyl)essigsäure-2-äthylhexylester

7) $X\text{-}O\text{-}CH_2\text{-}CH_2\text{-}O\text{-}X$, $Y\text{-}O\text{-}CH_2\text{-}CH_2\text{-}O\text{-}Y$

8) $(X\text{-}O\text{-}CH_2)_2\text{-}CH\text{-}O\text{-}X)$, $(Y\text{-}O\text{-}CH_2)_2\text{-}CH\text{-}O\text{-}Y$

9) $(X\text{-}O\text{-}CH_2)_4\text{-}C$, $(Y\text{-}O\text{-}CH_2)_4\text{-}C$

10) $(X\text{-}O\text{-}CH_2\text{-}CH_2)_2\text{-}O$, $(Y\text{-}O\text{-}CH_2\text{-}CH_2)_2\text{-}O$

11) $X\text{-}O\text{-}CH_2\text{-}C(CH_3)_2\text{-}CH_2O\text{-}X$, $Y\text{-}O\text{-}CH_2\text{-}C(CH_3)_2\text{-}CH_2\text{-}O\text{-}Y$

12) $X\text{-}O\text{-}CH_2\text{-}CH_2\text{-}O\text{-}CH_2\text{-}CH_2\text{-}O\text{-}CH_2\text{-}CH_2\text{-}O\text{-}X$, $Y\text{-}O\text{-}CH_2\text{-}CH_2\text{-}O\text{-}CH_2CH_2\text{-}O\text{-}CH_2\text{-}CH_2\text{-}O\text{-}Y$

13) $CH_3\text{-}C(CH_2\text{-}O\text{-}X)_3$, $CH_3\text{-}C(CH_2\text{-}O\text{-}Y)_3$

14) $CH_3CH_2\text{-}C(CH_2\text{-}O\text{-}X)_3$, $CH_3CH_2\text{-}C(CH_2O\text{-}Y)_3$

15) $H_{25}C_{12}N\text{-}(CH_2\text{-}CH_2\text{-}O\text{-}X)_2$, $H_{25}C_{12}N\text{-}(CH_2\text{-}CH_2\text{-}O\text{-}Y)_2$

16) $H_{37}C_{18}N\text{-}(CH_2\text{-}CH_2\text{-}O\text{-}X)_2$, $H_{37}C_{18}N\text{-}(CH_2\text{-}CH_2\text{-}O\text{-}Y)_2$

17) Gemisch der 2,4,4,4-Tetrachlorbuttersäure-$C_{20}$-$C_{22}$-alkylamide.

In den Verbindungen 7 bis 16 bedeuten

$$X = \overset{O}{\overset{\|}{-C}}\text{-}CH(Cl)\text{-}CH_2\text{-}CCl_3 \text{ und}$$

$$Y = -\overset{O}{\overset{\|}{C}}-CH-(CH_2-CCl_3)_2.$$

Die Verbindungen der Formel I können nach an sich bekannten Methoden hergestellt werden, insbesondere dadurch, dass man eine Verbindung der Formel II

$$Cl_3C-CH_2-\overset{R_1}{\overset{|}{C}H}-COOH \qquad (II),$$

worin $R_1$ die oben angegebene Bedeutung hat oder ein reaktionsfähiges Derivat davon den üblichen Veresterungs-, Amidierungs- oder Versalzungsreaktionen unterzieht. Als Reaktionspartner eignen sich für Veresterungsreaktionen Alkohole der Formel $HO-R_6$, für Amidierungsreaktionen Amine der Formel $HN(R_3)R_4$ und für Versalzungsreaktionen Amine der Formel $R_3N(R_4)R_5$.

Eine weitere Möglichkeit um zu Produkten der Formel I, worin $R_1$ Cl und $R_2$ $-N(R_3)R_4$ oder $-OR_6$ ist, und die übrigen Symbole die oben erwähnte Bedeutung haben, besteht darin, dass man in an sich bekannter Weise ein Acrylsäurederivat der Formel $CH_2=CH-C(O)R_2$, wobei $R_2$ $-N(R_3)R_4$ oder $-OR_6$ ist, und $R_3$, $R_4$ und $R_6$ die oben angegebene Bedeutung haben, in Gegenwart eines Katalysators, gegebenenfalls in einem Lösungsmittel mit Tetrachlorkohlenstoff umsetzt. Es ist bekannt, dass sich für die Halogenierung z.B. $FeCl_2$, $FeCl_3$ oder insbesondere CuCl eignen.

Selbstverständlich gelangt man auch durch übliche Umesterungs- bzw. Umamidierungs- oder Umsalzungsreaktionen zu den gewünschten Produkten.

2,4,4,4-Tetrachlorbuttersäure ist eine bekannte Verbindung und kann z.B. gemäss der israelitischen PS-Nr. 18771=C.A., 63, 13089e (1965) hergestellt werden, währenddem Bis-(2',2',2'-trichloräthyl)-essigsäure analog dem für das Säurechlorid im Beispiel 2 beschriebenen Verfahren aus Dichloressigsäure und 1,1-Dichloräthylen hergestellt werden kann. Sowohl die freie Säure und deren Chlorid sind neue Verbindungen und eignen sich z.B. als Zwischenprodukte zur Herstellung der Verbindungen der Formel I, worin $R_1$ $-CH_2-CCl_3$ bedeutet und $R_2$ die oben angegebene Bedeutung hat.

Die für die Umsetzung verwendeten Alkohole und Amine sind seit langem bekannte Verbindungen und vielfach handelsüblich.

Die Verbindungen der Formel I wirken schon in sehr geringen Mengen als Hochdruck-Zusätze in Schmiermitteln. So zeigen mineralische und synthetische Schmieröle, sowie deren Gemische, welche mit 0,001 bis 5 Gew.-%, bezogen auf das Schmiermittel, und vorzugsweise 0,02 bis 3% einer Verbindung der Formel I ausgestattet sind, ausgezeichnete Hochdruck-Schmiereigenschaften, welche durch stark reduzierte Abnutzungserscheinungen der zu schmierenden Reibpartner deutlich werden. Die in Frage kommenden Schmiermittel sind dem Fachmann geläufig und z.B. im "Schmiermittel Taschenbuch" (Hüthig Verlag, Heidelberg, 1974) beschrieben.

Die Schmierölformulierung kann zusätzlich noch andere Additive enthalten, die zugegeben werden, um gewisse Grundöleigenschaften zu verbessern, wie Antioxidantien, Metallpassivatoren, Rostinhibitoren, Viskositätsindex-Verbesserer, Stockpunkterniedriger, Dispersants/Detergents und andere Verschleissschutz-Additive.

0004957

Beispiele für Antioxidantien sind:

(a) Alkylierte und nicht-alkylierte aromatische Amine und Mischungen davon, z.B.: Dioctyldiphenylamin, Mono-t-octylphenyl-α- und -β-naphthylamine, Phenothiazin, Dioctylphenothiazin, Phenyl-α-naphthylamin, N,N'-Di-sec.butyl-p-phenylendiamin.

(b) Sterisch gehinderte Phenole, z.B. 2,6-Di-tert.butyl-p-cresol, 4,4'-Bis-(2,6-diisopropylphenol), 2,4,6-Triisopropylphenol, 2,2'-Thio-bis-(4-methyl-6-tert.butylphenol), 4,4'-Methylen-bis-(2,6-di-tert.butylphenol).

(c) Alkyl-, Aryl- oder Alkaryl-phosphite, z.B.: Trinonylphosphit, Triphenylphosphit, Diphenyldecylphosphit.

(d) Ester von Thiodipropionsäure oder Thiodiessigsäure, z.B.: Dilaurylthiodipropionat oder Dioctylthiodiacetat.

(e) Salze von Carbamin- und Dithiophsophor-säuren, z.B.: Antimon-diamyldithiocarbamat, Zink-diamyldithiophosphat.

(f) Kombinationen von zwei oder mehr Antioxidantion der obigen, z.B.: ein alkyliertes Amin und ein sterisch gehindertes Phenol.

Beispiele für Metallpassivatoren sind:

(a) für Kupfer, z.B. Benzotriazol, Tetrahydrobenzotriazol, 2-Mercaptobenzothiazol, 2,5-Dimercaptothiadiazol, Salicyliden-propylendiamin, Salze von Salicylaminoguanidin,

(b) für Blei, z.B. Sebacinsäurederivate, Chinizarin, Propylgallat,

(c) Kombination von zwei oder mehr der obigen Additive.

Beispiele für Rost-Inhibitoren sind:

(a) Organische Säuren, ihre Ester, Metallsalze und Anhydride, z.B.:
N-Oleoyl-sarcosin, Sorbitan-mono-oleat, Bleinaphthenat, Dodecenylbernsteinsäure-anhydrid.

(b) Stickstoffhaltige Verbindungen, z.B.:

I. primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen z.B.:
Substituierte Imidazoline und Oxazoline.

(c) Phosphorhaltige Verbindungen, z.B.:
Aminsalze von Phosphorsäurepartialester,

(d) Schwefelhaltige Verbindungen, z.B.:
Barium-dinonylnaphthalin-sulfonate, Calciumpetroleumsulfonate,

(e) Kombinationen von zwei oder mehr der obigen Additive.

Beispiele für Viskositätsindex-Verbesserer sind z.B.:
Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:
Polymethacrylate, alkylierte Naphthalinderivate.

Beispiele für Dispersants/Detergents sind z.B.:
Polybutenylbernsteinsäure-imide, Polybutenylphosphonsäurederivate, überbasische Magnesium-, Calcium- und Bariumsulfonate und -phenolate.

Beispiele für andere Verschleissschutz-Additive sind z.B.:
Schwefel und/oder Phosphor und/oder Halogen enthaltende Verbindungen, wie geschwefelte vegetabilische Oele, Zinkdialkyldithiophosphate, Tritolylphosphat, chlorierte Paraffine, Alkyl- und Aryldisulfide.

Die folgenden Beispiele erläutern die Erfindung.

0004957

Beispiel 1

226 g (1 Mol) 2,4,4,4-Tetrachlorbuttersäure, 600 g Thionylchlorid und 1 ml N,N-Dimethylformamid werden während 2 Stunden auf 50°C und während 2 Stunden auf 75°C erwärmt. Nach dem Abdampfen des überschüssigen Thionylchlorids wird der Rückstand destilliert. Man erhält 227,6 g (93% d. Th.) 2,4,4,4-Tetrachlorbuttersäurechlorid; Sdp. 90-91°C/15 mm Hg.

Beispiel 2

147,4 g (1 Mol) Dichloressigsäurechlorid, 291,8 g (3 Mol) 1,1-Dichloräthylen, 300 ml Acetonitril und 3,0 g Kupfer(I)chlorid werden in einem Autoklaven während 6 Stunden auf 160° erhitzt. Die Reaktionslösung wird hierauf filtriert, eingedampft und destilliert. Die bei Sdp. 160-165°C/13 Torr überdestillierende Fraktion wird aufgefangen. Man erhält 182,1 g (53% d. Th.) Bis-(2',2',2'-trichloräthyl)-essigsäurechlorid als schwach gelbe Flüssigkeit.

Beispiel 3

191,0 g Primen 81-R (Gemisch primärer $C_{12}$-$C_{15}$ t.-Alkylamine, Fa. Röhm und Haas, USA) werden in 500 ml Eisessig, 500 ml Wasser und 200 g Natriumacetat kristallisiert gelöst. Bei 0-5°C werden hierauf 244 g 2,4,4,4-Tetrachlorbuttersäurechlorid zugetropft. Man lässt die Temperatur des Reaktionsgemisches innerhalb von 2 Stunden auf Raumtemperatur steigen, setzt dann 2000 ml Wasser zu extrahiert mit 2000 ml Toluol. Die Toluolphase wäscht man mit einer 10%igen wässrigen Lösung von Natriumhydrogencarbonat und anschliessend mit Wasser. Nach dem Abdestillieren des Toluols im Vakuum erhält man 352,0 g (83% d. Th.) eines hellgelben dickflüssigen Oels, welches ein Gemisch der $C_{12}$-$C_{15}$ Alkylamide der 2,4,4,4-Tetrachlorbuttersäure (Additiv 1) darstellt.

## Beispiel 4

Wird in Beispiel 3 das 2,4,4,4-Tetrachlorbuttersäurechlorid durch 341,2 g Bis-(2',2',2'-trichloräthyl)-essigsäurechlorid ersetzt und sonst gleich verfahren, so erhält man 390,0 g eines hellgelben zähflüssigen Oels, welches ein Gemisch der $C_{12}$-$C_{15}$ Alkylamide der Bis-(2',2',2'-trichloräthyl)-essigsäure (Additiv 2) darstellt.

## Beispiel 5

30,4 g (0,33 Mol) Glycerin wasserfrei werden langsam mit 244,4 g (1,0 Mol) 2,4,4,4-Tetrachlorbuttersäurechlorid (hergestellt nach Beispiel 1) versetzt. Nach dem Abklingen der exothermen Reaktion erwärmt man das Reaktionsgemisch während 5 Stunden auf 120-125°. Anschliessend wird im Hochvakuum destilliert und die bei Sdp. 155-157°C/0,1 Torr siedende Fraktion aufgefangen. Man erhält 182,6 g einer fast farblosen viskosen Flüssigkeit der Formel

0004957

$$Cl_3C—CH_2—CHCl—COO—CH_2$$
$$Cl_3C—CH_2—CHCl—COO—CH$$
$$Cl_3C—CH_2—CHCl—COO—CH_2$$

(Additiv 3).

Beispiel 6

270,5 g 1-Octadecanol (1,0 Mol) werden als Schmelze bei 60°C langsam mit 244,4 g 2,4,4,4-Tetrachlorbuttersäurechlorid (1,0 Mol) versetzt und hierauf während 5 Stunden auf 120-125° erhitzt. Man erhält 470,5 g einer sehr viskosen hellgelben Flüssigkeit, die im Hochvakuum bei 0,1 Torr und 220°C nicht destillierbar ist. Das Produkt ist 2,4,4,4-Tetrachlorbuttersäure-octadecylester (Additiv 4).

Beispiel 7

Wird in Beispiel 5 das Glycerin durch 220,3 g p-Nonylphenol (Fa. Montedison) ersetzt und sonst gleich verfahren, so erhält man 460,3 g 2,4,4,4-Tetrachlorbuttersäure-p-nonylphenylester, in Form eines braunen Oels, das im Hochvakuum bei 0,1 Torr zwischen 160-170°C destillierbar ist (Additiv 5).

Beispiel 8

Wird in Beispiel 5 das Glycerin durch 75,1 g (0,5 Mol) Triäthylenglykol ersetzt und sonst gleich verfahren, so erhält man nach dem Erhitzen im Hochvakuum bei 0,1 Torr auf 180°C 230,7 g eines hellbraunen, sehr zähflüssigen Oels der Formel

$$(Cl_3C\text{-}CH_2\text{-}CHCl\text{-}COO\text{-}CH_2\text{-}CH_2\text{-}O\text{-}CH_2)_2\text{-}$$

(Additiv 6).

Beispiel 9

Wird in Beispiel 5 das Glycerin durch 59,1 g (0,5 Mol) 1,6-Hexandiol ersetzt und sonst gleich verfahren, so erhält man nach dem Abdestillieren geringer Mengen von Ausgangsmaterial bei 180° im Hochvakuum (0,1 Torr) 210,8 g eines schwach geleben sehr zähflüssigen Oels der Formel

$$(Cl_3C\text{-}CH_2\text{-}CHCl\text{-}COO\text{-}CH_2\text{-}CH_2\text{-}CH_2)_2\text{-}$$

(Additiv 7).

Beispiel 10

150,0 g 2-Aethylhexanol (1,15 Mol) werden langsam mit 341,2 g Bis-(2',2',2'-trichloräthyl)-essigsäurechlorid (1,0 Mol) (aus Beispiel 2) versetzt und hierauf 6 Stunden auf 120° erhitzt. Anschliessend wird im Vakuum bei

12 Torr und 100-120° das überschüssige 2-Aethylhexanol abdestilliert. Es bleiben 434,1 g eines hellgelben dickflüssigen Oels zurück, das im Hochvakuum (0,1 Torr) nicht destillierbar ist. Das Produkt ist der Bis-(2',2',2'-Trichloräthyl)-essigsäure-(2-äthylhexyl)-ester (Additiv 8).

Beispiel 11

Wird in Beispiel 10 das Bis-(2',2',2'-trichloräthyl)-essigsäurechlorid durch 244,4 g (1 Mol) 2,4,4,4-Tetrachlorbuttersäurechlorid ersetzt und sonst gleich verfahren, so erhält man 330,2 g 2,4,4,4-Tetrachlorbuttersäure-(2-äthylhexyl)-ester, in Form eines fast farblosen viskosen Oels, das bei 114-116°C/2 Torr siedet und der Formel

$$Cl_3C-CH_2-CHCl-COO-CH_2-\underset{}{\overset{C_2H_5}{\overset{|}{CH}}}-(CH_2)_3-CH_3$$

entspricht (Additiv 9).

Beispiel 12

6,6 g (0,03 Mol) 2,4,4,4-Tetrachlorbuttersäure werden in 50 ml Toluol gelöst und unter Rühren mit 5,8 g (0,03 Mol) Primene 81-R (Gemisch primärer $C_{12}$-$C_{15}$ t.Alkylamine, Rohm und Haas, USA) versetzt. Das Lösungsmittel wird unter reduziertem Druck vollständig abdestilliert. Man erhält so ein gelbliches, in Hexan bzw. Mineralöl leicht lösliches durchsichtiges Oel, welches ein Gemisch der Dodecyl-, bis Pentadecylammoniumsalze der 2,4,4,4-Tetrachlorbuttersäure ist (Additiv 10).

Ersetzt man in diesem Beispiel das Primene 81-R durch eine äquivalente Menge Diisooctylamin oder Diisotridecylamin, so erhält man bei sonst gleicher Arbeitsweise die entsprechenden Ammoniumsalze als gelbliche, durchsichtige Oele, nämlich das Diisooctylammoniumsalz der 2,4,4,4-Tetrachlorbuttersäure (Additiv 11) und das Diisotridecylammoniumsalz der 2,4,4,4-Tetrachlorbuttersäure (Additiv 12).

Beispiel 13

Mit dem Shell-Vierkugel-Apparat wurden folgende Werte bestimmt: (Tentative method IP 239/69, Extreme pressure and wear lubricant test for oils and greases, four ball-machine).

1) I.S.L. = Initial Seizure Load: Das ist die Last, bei der der Oelfilm innerhalb einer Belastungsdauer von 10 Sekunden zusammenbricht.

2) W.L. = Weld Load (Schweisslast). Das ist die Last, bei der die 4 Kugeln innerhalb von 10 Sekunden zusammenschweissen.

3) W.S.D. = Wear Scar Diameter in mm: Das ist der mittlere Verschleissdurchmesser bei einer Belastung von 40 kg während 1 Stunde.

Als Basisöl wurde Vitrea 41 (Handelsbezeichnung der Firma Shell) verwendet.

Table 1

| Additiv Nr. | Konz. in Gew.-% | ISL (kg) | WL (kg) | WSD (mm) |
|---|---|---|---|---|
| Keines | - | 60 | 160 | 1,1 |
| 1 | 1% | 80 | 400 | 0,8 |
| 3 | 1% | 130 | 270 | 0,7 |
| 4 | 1% | - | >200 | 0,6 |
| 5 | 1% | 100 | 230 | 0,9 |
| 6 | 1% | - | >200 | 0,5 |
| 7 | 1% | - | >200 | 0,5 |
| 8 | 1% | 110 | 250 | 0,8 |
| 9 | 1% | 110 | 250 | 0,8 |
| 10 | 1% | 160 | 500 | 0,8 |
| 11 | 1% | - | >200 | 1,1 |
| 12 | 1% | - | >200 | 0,5 |

Beispiel 14

Die aussergewöhnlichen Lasttrageeigenschaften der erfindungsgemässen Schmierstoffzusätze zeigen sich bei der Prüfung im FZG-Zahnradverspannungsprüfstand.

Zu diesem Zweck wurden Mischungen der erfindungsgemässen Additive in einem nicht-legierten mineralischen Schmieröl (Viskosität: 20cSt/50°C) hergestellt und mit der FZG-Maschine nach DIN 51354 (Normaltest A/8.3/90 geprüft). Vergleichsweise wurde auch das nicht-legierte mineralische Schmieröl ohne Zusatz mit der FZG-Maschine geprüft.

Die Ergebnisse dieser Untersuchungen sind in der nachstehenden Tabelle 2 zusammengestellt.

Tabelle 2

| Test Nr. | Additiv Nr. | Konzentration Gew. % | Schadenslaststufe |
|---|---|---|---|
| 1 | kein | - | 6-7 |
| 2 | Chlorparaffin 50 *) | 0,5 | 7 |
| 3 | 1 | 0,5 | 11 |
| 4 | 3 | 0,5 | 11 |
| 5 | 12 | 0,5 | 12 |

Man ersieht aus diesen Ergebnissen die hervorragenden Lasttrageeigenschaften der erfindungsgemässen Verbindungen.

*) ex Hoechst ®

## Patentansprüche

1. Verbindungen der allgemeinen Formel I

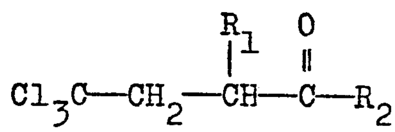

(I) ,

worin

$R_1$ Chlor oder $-CH_2-CCl_3$ ist, und

$R_2$ $-N(R_3)R_4$, $-OH \cdot N(R_3)(R_4)R_5$ oder $-OR_6$ ist, wobei

$R_3$ $C_1-C_{24}$ Alkyl ist, welches gegebenenfalls ein- oder mehrmals mit einem Sauerstoffatom unterbrochen sein kann, und

$R_4$ und $R_5$ unabhängig voneinander Wasserstoff oder $C_1-C_{24}$ Alkyl bedeuten, wobei $R_3$ und $R_4$ zusammen mindestens 8 C-Atome besitzen, und

$R_6$ $C_8-C_{20}$ Alkyl, Phenyl oder $C_7-C_{15}$ Alkylphenyl, eine Gruppe der Formel $-(A)R_7$ oder $-CH_2CH_2-N(R_{10})-CH_2CH_2-O-C(O)-CH(R_1)CH_2CCl_3$ ist, worin

$R_7$ einen Rest der Formel $-(R_8)C(R_9)-O-C(O)-CH(R_1)-CH_2-CCl_3$ bedeutet, wobei

$R_8$ und $R_9$ unabhängig voneinander Wasserstoff, Methyl oder Aethyl sind, und $R_1$ die oben angegebene Bedeutung hat, und

A eine gegebenenfalls mit einem oder zwei Resten $R_7$ oder einer oder zwei Methyl- oder Aethylgruppen substituierte $C_1-C_7$ Alkylengruppe ist, wel-

che gegebenenfalls durch -O- unterbrochen sein kann, und

$R_{10}$ $C_4$-$C_{20}$ Alkyl bedeutet.

2. Verbindungen gemäss Anspruch 1 der Formel I, worin

$R_1$ Chlor oder $-CH_2-CCl_3$ bedeutet, und

$R_2$ $-N(R_3)R_4$, $-OH \cdot N(R_3)(R_4)R_5$ oder $-OR_6$ ist, und

$R_3$ $C_4$-$C_{18}$ Alkyl ist, und

$R_4$ und $R_5$ unabhängig voneinander Wasserstoff oder $C_4$-$C_{18}$ Alkyl bedeuten, wobei $R_3$ und $R_4$ zusammen mindestens 8 C-Atome besitzen, und

$R_6$ $C_8$-$C_{18}$ Alkyl oder $C_7$-$C_{15}$ Alkylphenyl oder eine Gruppe der Formel $-(A)R_7$ ist, worin

$R_7$ einen Rest der Formel $-(R_8)C(R_9)-O-C(O)-CH(R_1)-CH_2-CCl_3$ bedeutet, wobei

$R_8$ und $R_9$ unabhängig voneinander Wasserstoff, Methyl oder Aethyl sind, und $R_1$ die oben angegebene Bedeutung hat und

eine gegebenenfalls mit einem oder zwei Resten $R_7$ oder einer oder zwei Methyl- oder Aethylgruppen substituierte $C_1$-$C_5$ Alkylengruppe oder eine 3,6-Dioxaheptamethylengruppe bedeutet.

3. Verbindungen gemäss Anspruch 1 der Formel I, worin

$R_1$ Chlor oder $-CH_2-CCl_3$ bedeutet, und

$R_2$ $-N(R_3)(R_4$, $-OH \cdot N(R_3)(R_4)R_5$ oder $-OR_6$ ist, und

$R_3$ $C_4$-$C_{18}$ Alkyl ist, und

$R_4$ und $R_5$ unabhängig voneinander Wasserstoff oder $C_4$-$C_{18}$ Alkyl bedeuten, wobei $R_3$ und $R_4$ zusammen mindestens 8 C-Atome besitzen, und

$R_6$ $C_8$-$C_{18}$ Alkyl, $C_7$-$C_{15}$ Alkylphenyl oder eine Gruppe der Formel $-(A)R_7$ ist, worin

$R_7$ einen Rest der Formel $-CH_2-O-C(O)-CH(R_1)-CH_2-CCl_3$ bedeutet, wobei $R_1$ die oben angegebene Bedeutung hat und

A $C_1-C_5$ Alkylen, 3,6-Dioxaheptamethylen oder eine der Gruppen der Formeln $-CH(R_7)-$, $-CH_2CH_2CH(R_7)-CH_2-$ oder $-CH_2-C(R_7)_2-$ bedeutet, worin $R_7$ die oben angegebene Bedeutung hat.

4. Verbindungen gemäss Anspruch 1, der Formel I, worin $R_1$ Cl ist.

5. Verbindungen gemäss Anspruch 1 der Formel I, worin $R_1$ $-CH_2-CCl_3$ ist,

6. Verbindungen gemäss Anspruch 1 der Formel I, worin $R_2$ $-N(R_3)R_4$ ist.

7. Verbindungen gemäss Anspruch 1 der Formel I, worin $R_2$ $-OH\cdot N(R_3)(R_4)R_5$ ist.

8. Verbindungen gemäss Anspruch 1 der Formel I, worin $R_2$ $-OR_6$ ist.

9. Stoffzusammensetzungen bestehend aus einem Schmiermittel und einer Verbindung der Formel I gemäss Anspruch 1.

10. Verwendung der Verbindungen der Formel I gemäss Anspruch 1 als Schmiermittel-Additive.

11. Bis-(2',2',2'-trichloräthyl)-essigsäure.

12. Bis-(2',2',2'-trichloräthyl)-essigsäurechlorid.